Europäisches Patentamt

European Patent Office

Office européen des brevets

(19)

(11) **EP 0 691 560 A1**

(12) **EUROPEAN PATENT APPLICATION**

(43) Date of publication:
**10.01.1996 Bulletin 1996/02**

(51) Int Cl.6: **G02C 1/00**, G02C 7/02,
G02C 7/16

(21) Application number: **95110558.4**

(22) Date of filing: **06.07.1995**

(84) Designated Contracting States:
**DE FR GB**

(30) Priority: **06.07.1994 KR 9416404**

(71) Applicant: **Min, Byung-Moo**
**Taejeon-city (KR)**

(72) Inventor: **Min, Byung-Moo**
**Taejeon-city (KR)**

(74) Representative: **Patentanwälte**
**Beetz - Timpe - Siegfried Schmitt-Fumian - Mayr**
**D-80538 München (DE)**

(54) **Lens for diplopia and amblyopia and glasses using the same**

(57)     The present invention relates to a lens (7) for a diplopia and an amblyopia and glasses (12) using the same for remedial treatment of a patient suffering from diplopia and amblyopia by which to increase an effective treatment result and to enhance satisfaction of a wearer by compensating drawbacks in that a conventionally used occluder has caused the wearer inconvenience and the wearer has avoided wearing the occluder due to deficiency in the esthetic sense.

CORRIGENDUM issued on 14.02.96

EP 0 691 560 A1

# FIG.7

Printed by Jouve (FR), 18, rue Saint-Denis, 75001 PARIS

**Description**

BACKGROUND OF THE INVENTION

FIELD OF THE INVENTION

The present invention relates to a lens specially manufactured for treatment of a diplopia and an amblyopia and glasses using the same, and more particularly to a lens for remedial treatment of a diplopia and an amblyopia and glasses using the same by which to increase an effective treatment result and to enhance satisfaction of a wearer by compensating drawbacks in that a conventionally used occluder has caused the wearer inconvenience and the wearer has avoided wearing the occluder due to deficiency in the esthetic sense.

DESCRIPTION OF THE PRIOR ART

Amblyopia is defined as dimness of sight in one eye or in both eyes without apparent organic defect and most of the cases, it occurs in one eye. Amblyopia is known to be caused by interdiction of an appropriate visual impetus indispensible for development of eye sight in the early ages by strabismus, anisometropia, heavy abnormal refraction and the like. However it is a kind of disease curable to normalcy when treated in an early stage. The ratio of people suffering from this disease is roughly 2-5 percent of the total population.

Diplopia is defined as a pathological condition of vision in which a single object appears double caused by a extraocular muscle paralysis and the like.

Diplopia is known to be originated from generation of strabismus caused by nervous paralysis of eye muscle resulting from trauma circulatory disorders such as diabetes, high blood pressure, trauma and the like, and most of the cases, it is possible to recover from diplopia within 6 months the disease has occurred.

In the case of the amblyopia, the eye sight can be improved by an appropriate treatment until a period of age 9 to age 10.

It is mostly known in the prior art that a good eye (a normal eye of good eye sight) is covered by an occluder to thereby cause the same not to see, so that only the eye of amblyopia can see for improvement of the same.

Even in the case of diplopia, it has been a customary method for a patient of diplopia to wear the occluder for improvement of diplopia.

However, there is a problem or inconvenience in the occluding method or covering method utilizing the occluder in that the occluder should be periodically replaced due to secretion collecting at an inside surface of the occluder (portion where the eye contacts), and especially the case is worsened when the occluder should be worn for a lifetime in case of diplopia caused by a traumatic injury because symptom of diplopia remains even after the surgery.

There is another problem in that the occluder is frequently removed for reasons of its external appearance and lack of esthetic look on the face especially in the case of a child patient, thereby decreasing remedial cooperation from the patient and lowering a curative effect.

There is still another problem in that complaints of itchy symptom are occasionally appealed because of dermatitis on a facial area caused by adhesive tape for attaching the occluder to the face.

SUMMARY OF THE INVENTION

The present invention is therefore disclosed to solve the afore-referenced problems and it is an object of the present invention to provide a lens for a diplopia and an amblyopia and glasses using the same, by which no esthetic defects appear ostensibly so that an eye through the glasses using the lens according to the present invention look like a normal eye and can be seen clearly from outside of the glasses as if worn by regular glasses, and a wearer thereof can hardly see objects in the outside to thereby obtain an effect of occluding normal eyes replacing the conventional occluder, and to improve an eye sight of a patient suffering from the diplopia or the amblyopia.

In accordance with the object of the present invention, there is provided a lens for a diplopia and an amblyopia and glasses using the same, the lens comprising a lens unit for being formed at one side thereof with a concave at an inside surface facing the eyeball and the lens unit having a non-reflecting multi-coated surface thereon.

BRIEF DESCRIPTION OF THE DRAWINGS

For fuller understanding of the nature and objects of the invention, reference should be made to the following detailed description taken in conjunction with the accompanying drawing in which:

Figure 1 is a conventional occluder ( eye patch ) on the market;

Figure 2 is a hand-made occluder made of a conventionally used gauze;

Figure 3 is a plan of glasses using the lens according to the present invention;

Figure 4 is an enlarged sectional view of "A" part in Figure 3;

Figure 5 is a schematic diagram for illustrating a case where an eyeball of the wearer of the glasses using the lens according to the present invention is seen from the outside thereof;

Figure 6 is a schematic diagram for illustrating a case where the wearer sees the objects in the outside;

Figure 7 is a perspective view for illustrating a state of use of the glasses according to the present invention; and

Figure 8 is a plan for illustrating a state of use of the glasses according to the present invention.

## DETAILED DESCRIPTION OF THE PREFERRED EMBODIMENTS

The preferred embodiment of the present invention will be described in detail with reference to the accompanying drawing.

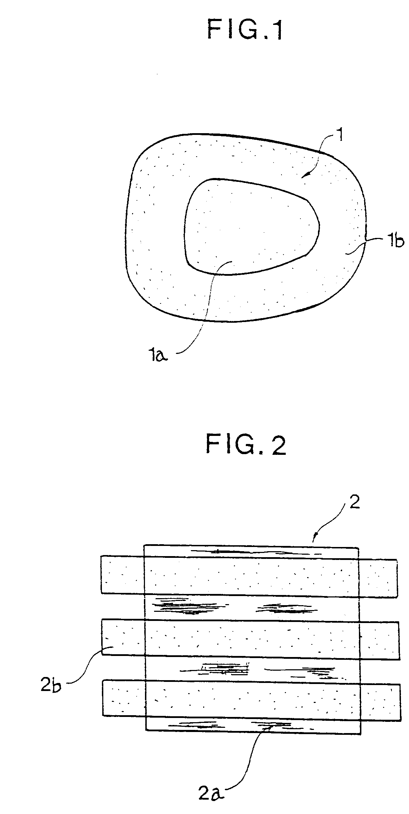

Figure 1 is a conventional occluder ( eyepatch ) on the market, where the same includes a gauze la and a tape 1b.

Figure 2 is an eye patch made of a conventionally used gauze, where the gauze 2a covers the eye and the eye patch is attached to a face with a tape 2b.

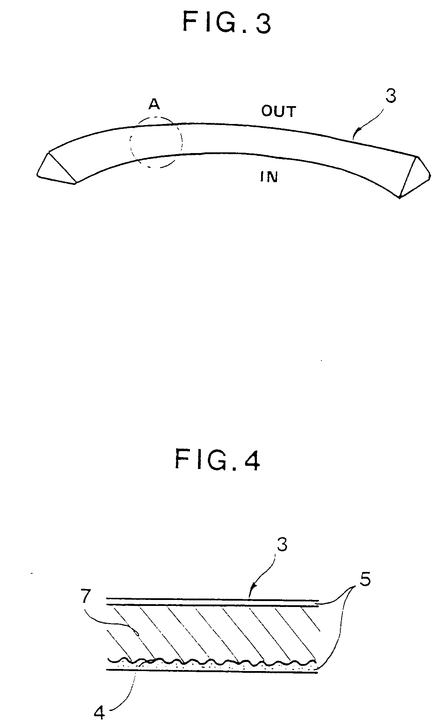

Figure 3 and 4 illustrate an embodiment of the present invention, where the embodiment includes, from an inside thereof (in order words, from the side of the eyeball), a non-reflecting multi-coated surface 5 and a lens unit 7 having a concaved surface 4.

In order to produce the lens thus constructed, glass or plastic material of no degree (diopter) is used, and the lens unit 7 thereof is processed at an inside surface thereof (in other words, at an eyeball side) utilizing diamond particles, sanding machine, molding machine, chemical substance and the like to thereby cause the lens to have a finely processed concave surface.

In the present embodiment, although the concaved surface in processed to form at a side facing the eyeballs, it should be possible that the concaved surface is formed at a side facing an outside surface of the lens 7.

Then, the concave surface and an outside surface of the lens unit 7 are multi-coated with non-reflecting material such as oxidized zirconium or oxidized silicon SiO2, to thereby finish the lens.

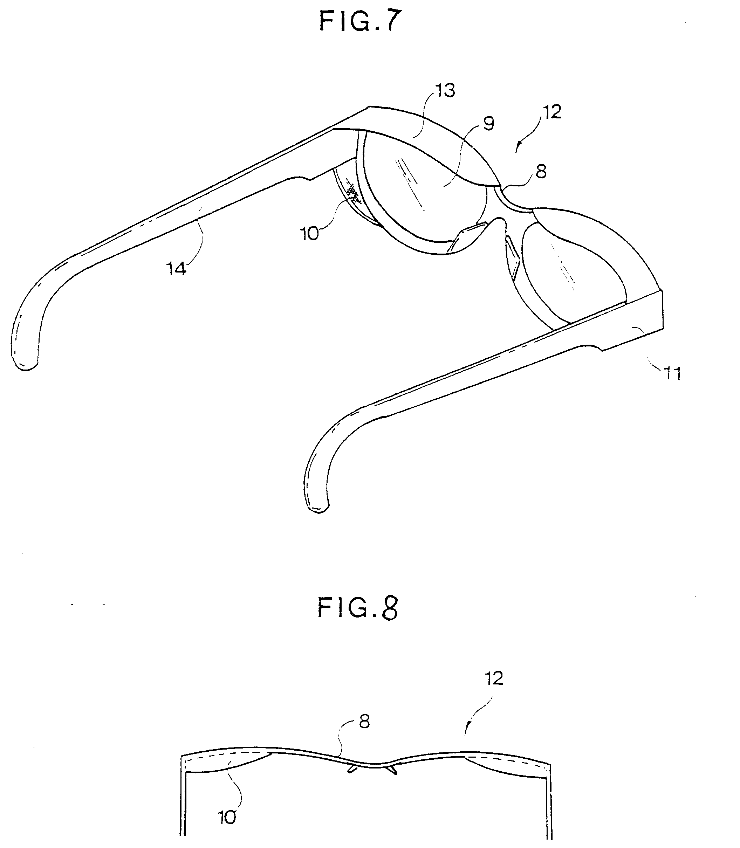

Figures 7 and 8 are a perspective and a plan view respectively according to the present invention, where the glasses are fitted at one side thereof with the lens according to the present invention and are equipped at the other side thereof with a lens made of an ordinary glass or plastic material of no degrees.

Furthermore, as illustrated in Figure 7, an upper side blocking unit 13 and a lower side blocking unit 10 are arranged at a periphery of a temple, and a lateral blocking unit 11 of a predetermined width is provided at a connecting portion to the temple, so that no object can be seen by patient to thereby increase a treatment effect.

It should be apparent that the upper side blocking unit 13 and the lower side blocking unit 10 are integrally manufactured with a frame, or the frame is manufactured in the first place to thereafter be attached by the blocking units 10 and 13. Size of the blocking unit should be large enough to keep the wearer from seeing an outside object squinting.

The lateral blocking unit 11 can be integrally manufactured with the temple or can be omitted altogether, or can be extended all the way to a side of the temple.

Meanwhile, there is generated a scattering of light on the concaved surface of the lens, thereby causing an image of the object to be obscured, so that the outside object cannot be seen at an eyeball side but the eyeball can be widely seen from the outside.

The principle of this effect will now be described.

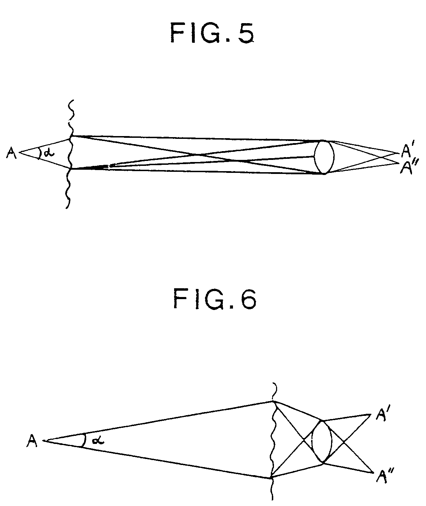

As illustrated in Figure 5, if the point A is situated near the concaved surface, even though an image of the point A is scattered on the concaved surface, a distance between A' and A" (A' or A" being a place where the image is created on a retina) is shortened because the retina of the eyeball for receiving the image is distantly located. Therefore, the image on the point A is seen relatively clear.

The afore-referenced case in identical to a case where the eyeball of the wearer behind the concaved surface is seen, which means ofter all that the eyeball of the wearer can be clearly seen even though there exists the concaved surface.

Conversely, as illustrated in Figure 6, if the point A is distanced from the concaved surface, the image of the point A is scattered after passing the concaved surface, and the image is created on the retina of the eyeball near the concaved surface.

At this location, because the distance between A' and A" where the image is made on the retina is far due to retina of the eyeball for receiving the image being so close to the concaved surface, so that it is difficult to distinguish the image.

This case is identical to a case where the wearer sees an outside object through the concaved surface, which means that the outside object cannot be seen by the wearer.

According to this theory, it should be further advisable that the concaved surface rather be formed on an inside of the lens than on an outside of the lens and that flat lens rather be closed to the eyeball, so it will be more effective.

Furthermore, both inside and outside surface of the lens are multi-coated with non-reflecting material.

In case a refractive index n of a lens is 1.5, reflection factor r' at an outside surface of the lens can be given as,

$$\left(\frac{1-n}{1+n}\right)^2 \times 100\% = 4\%$$

where, n=1.5.

At this time, reflection factor r" at an inside surface of the lens is also 4%. The lens looks obscured due to reflection and scattering on the inside surface caused by the concaved surface of the lens when viewed toward the eyes from outside of the lens.

The non-reflection multi-coating can eliminate this kind of reflection, thereby allowing a clear view of the eyeball from outside of the lens.

If necessary, a certain tint can be inserted into the lens.

When the amblyopia is treated with glasses of the lens thus described, a lens of no degree in case of no refraction, or a lens of an appropriate degree in case of refraction is worn by the eye of the amblyopia, and the lens according to the present invention is worn by the eye of normal eyesight.

Furthermore, in order to treat the diplopia, when both eyes have the same eyesight, any eye be worn by the lens of the present invention, and when the eyesight is different between the eyes, the lens according to the present invention is worn by the eye of bad eyesight.

In both cases described in the above, the lens according to the present invention prevents the eye from seeing the objects, thereby causing the wearer thereof to obtain the same effect as wearing the eye patch or occluder.

Furthermore, the eye wearing the lens of the present invention is well viewed from the outside, so that esthetic defects of the conventional eye patch can be eliminated to thereby increase the treatment cooperation from the patient.

As illustrated in Figure 7 and 8, because the frame and temple are respectively formed with blocking units, the wearer of the glasses cannot squint to see the objects, so that a blindfolding effect can be improved for further increase of the treatment result.

The width of the blocking unit should be wide enough to bar the wearer from squinting to see the objects. It should also be possible that the blocking unit is separately manufactured to thereafter be attached to the frame, or is integrally manufactured with the frame or the temple.

The treatment result by way of the glasses inserted with the lens according to the present invention for the patient suffering from the amblyopia or diplopia can be found as follows.

Amblyopia Treatment

Among 49 patients who have shown no remedial values against the amblyopia due to poor cooperation to the conventional occluder treatment, 35 patients (71.4%) have registered eye sight improvement of $2.54 \pm 1.43$ lines by Snellen acuity after three months of wearing the glasses inserted with the lens according to the present invention.

A group who has registered one line in the eye sight improvement was numbered 10 (20.4%), a group of 2 lines numbered 10 (20.4%), a group of 3 lines numbered 6 (12.2%), a group of 4 or more lines numbered 9 (18.2%) and a group who has shown no improvement in the eye sight was counted 14 (28.5%).

In a satisfaction test, a subjective satisfaction was 85.8% while an objective satisfaction was tallied at 95.2%, which represents that wearing of the glasses of the lens according to the present invention has provided an esthetic effect to both the patients and protectors thereof.

Degree of eye sight improvement according to the subjective satisfaction in a group who responded as "Very good", "good" and "so so" has respective eye sight improvement of $3.88 \pm 5.61$ lines (p<0.05), $0.95 \pm 095$ lines (p<0.01) and $0.67 \pm 0.86$ lines (p<0.05), whereas the degree of improvement in a group who responded as "bad" improvement of $0.43 \pm 0.67$ lines (p>0.05).

This means that the group of favorable responses has marked a significant improvement of eye sight compared with that of negative responses.

Degree of eye sight improvement per age group differs. By way of example, a group of less than 4 had an improvement of $2.67 \pm 2.3$ lines, a group of more than 4 to less than 7 had an improvement of $1.64 \pm 2.31$ lines.

In other words, the group of less than age 4 has shown the most outstanding improvement result (p<0.05).

Meanwhile, in the eye sight improvement with regard to causes leading to amblyopia, a group of strabismic amblyopia showed an improvement of $310 \pm 5.76$ lines, a group of anisometropic amblyopia had an improvement of $1.52 \pm 1.73$ lines and a group of visual deprivation amblyopia registered an improvement of $0.82 \pm 6.42$ lines.

In other words, groups of strabismic amblyopia and Anisometropic amblyopia represented outstanding improvements (p<0.05).

Diplopia Treatment

As a result of treatment to 10 (ten) adults who suffered from diplopia by having them wear the glasses of lens according to the present invention, the ten adults all were cured of the diplopia and all expressed satisfaction with regard to outward appearance involved in wearing the glasses.

As apparent from the foregoing, it can be seen that the glasses using the lens for diplopia and amblyopia according to the present invention has increased a treatment effect and improved a wearer's satisfaction thereof, and has made up for the weak points in that there have been inconveniences involved in wearing an eye patch or occluder conventionally used for the treatment of amblyopia or diplopia and cases of avoiding wearing the eye patch or the occluder due to esthetic deficiency, to thereby decrease curative value.

**Claims**

1. A lens for diplopia and amblyopia, the lens comprising a lens unit (7) for being formed at one side thereof with a concave (4), wherein the concave is formed with a non-reflecting multi-coated surface (5) thereon.

2. Glasses using the lens for diplopia and amblyopia as defined in claim 1, wherein one side thereof is inserted with the lens while the other side thereof is equipped with a conventional lens.

3. Glasses using the lens for diplopia and amblyopia as defined in claim 2, wherein a temple (8) thereof is arranged at an upper periphery thereof with an upper side blocking unit (13) and at a lower periphery thereof with a lower side blocking unit (10), and at a lateral side thereof with a lateral blocking unit (11).

4. Glasses using the lens for diplopia and amblyopia as defined in claim 3, wherein the blocking units (10, 11 and 13) are integrally formed with the temple (8).

5. Glasses using the lens for diplopia and amblyopia as defined in claim 3, wherein the lower side blocking unit (10) is integrally sunshaded up to a lateral surface unit of the temple (8).

6. Glasses using the lens for diplopia and amblyopia as defined in claim 3, wherein the lateral side blocking unit (11) is formed with an enlargement in width thereof at a joint between the temple (8 )and a frame (14 )thereof.

# FIG.1

# FIG.2

# FIG. 3

A        OUT        3

IN

# FIG. 4

3

7        5

4

FIG. 5

FIG. 6

## FIG.7

## FIG.8

EP 0 691 560 A1

**European Patent Office**

**EUROPEAN SEARCH REPORT**

Application Number
EP 95 11 0558

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | US-A-5 204 702 (A.W. SHAPIRO)<br>* column 2 - column 3, line 50 *<br>--- | 1,2 | G02C1/00<br>G02C7/02<br>G02C7/16 |
| A | FR-A-1 570 723 (L.L. DECROP)<br>* page 5, line 9 - line 24 *<br>--- | 3-6 | |
| A | US-A-5 264 877 (E.S. HUSSEY)<br>* column 1 - column 2, line 32 *<br>--- | 1,2 | |
| A | US-A-3 904 281 (A. JAMPOLSKY)<br>* column 1 - column 2 *<br>--- | 1,2 | |
| A | US-A-4 288 149 (C.D. CAMPBELL)<br>* column 2, line 30 - column 5, line 48 *<br>--- | 1 | |
| A | US-A-4 070 097 (R.M. GELBER)<br>* column 1, line 44 - column 2, line 21 *<br>----- | 1 | |
| | | | TECHNICAL FIELDS SEARCHED (Int.Cl.6)<br><br>G02C |

The present search report has been drawn up for all claims

| Place of search | Date of completion of the search | Examiner |
|---|---|---|
| THE HAGUE | 11 October 1995 | CALLEWAERT, H |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

10